(19) Europäisches Patentamt
European Patent Office
Office européen des brevets

(11) EP 0 764 632 B1

# (12) EUROPEAN PATENT SPECIFICATION

(45) Date of publication and mention of the grant of the patent:
**25.07.2001 Bulletin 2001/30**

(21) Application number: **96306850.7**

(22) Date of filing: **20.09.1996**

(51) Int Cl.[7]: **C07C 233/52**, C07C 235/42, C07C 235/66, C07C 255/54, C07C 323/63, C07C 311/08, C07D 295/18, C07D 213/82, C07D 255/04, C07D 333/34, A61K 31/165

(54) **Selective beta3 adrenergic agonists**

Selektive Beta3-adrenergische Agonisten

Agonistes sélectifs bêta3-adrénergiques

(84) Designated Contracting States:
**AT BE CH DE DK ES FI FR GB GR IE IT LI LU NL PT SE**
Designated Extension States:
**AL LT LV SI**

(30) Priority: **21.09.1995 US 4083**

(43) Date of publication of application:
**26.03.1997 Bulletin 1997/13**

(73) Proprietor: **ELI LILLY AND COMPANY**
**Indianapolis, Indiana 46285 (US)**

(72) Inventors:
- **Evrard, Deborah Ann**
  **Indianapolis, Indiana 46254 (US)**
- **McDonald, John Hampton**
  **Carmel, Indiana 46032 (US)**
- **Matthews, Donald Paul**
  **Indianapolis, Indiana 46250 (US)**
- **Rito, Christopher John**
  **Mooresville, Indiana 46158 (US)**
- **Shuker, Anthony John**
  **Indianapolis, Indiana 46208 (US)**
- **Bell, Michael Gregory**
  **Indianapolis, Indiana 46240 (US)**
- **Crowell, Thomas Alan**
  **Indianapolis, Indiana 46220 (US)**
- **Winter, Mark Alan**
  **Indianapolis, Indiana 46220 (US)**

(74) Representative: **Tapping, Kenneth George et al**
**Lilly Industries Limited**
**European Patent Operations**
**Erl Wood Manor**
**Windlesham Surrey GU20 6PH (GB)**

(56) References cited:

| | |
|---|---|
| **EP-A- 0 375 560** | **EP-A- 0 611 003** |
| **EP-A- 0 626 367** | **EP-A- 0 659 737** |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 764 632 B1

Printed by Jouve, 75001 PARIS (FR)

## Description

The present invention is in the field of medicine, particularly in the treatment of Type II diabetes and obesity. More specifically, the present invention relates to selective β3 adrenergic receptor agonists useful in the treatment of Type II diabetes and obesity.

Background of the Invention

The current preferred treatment for Type II, non-insulin dependent diabetes as well as obesity is diet and exercise, with a view toward weight reduction and improved insulin sensitivity. Patient compliance, however, is usually poor. The problem is compounded by the fact that there are currently no approved medications that adequately treat either Type II diabetes or obesity. The invention described herein is directed toward an effective and timely treatment for these serious diseases.

One therapeutic opportunity that has recently been recognized involves the relationship between adrenergic receptor stimulation and anti-hyperglycemic effects. Compounds that act as $\beta_3$ receptor agonists have been shown to exhibit a marked effect on lipolysis, thermogenesis and serum glucose levels in animal models of Type II (non-insulin dependent) diabetes.

The $\beta_3$ receptor, which is found in several types of human tissue including human fat tissue, has roughly 50% homology to the $\beta_1$ and $\beta_2$ receptor subtypes yet is considerably less abundant. The importance of the $\beta_3$ receptor is a relatively recent discovery since the aminoacid sequence of the human receptor was only elucidated in the late 1980's. A large number of publications have appeared in recent years reporting success in discovery of agents that stimulate the $\beta_3$ receptor. Despite these recent developments, there remains a need to develop a selective $\beta_3$ receptor agonist which has minimal agonist activity against the $\beta_1$ and $\beta_2$ receptors.

EP-A-375 560 discloses certain aryloxypropanolamines having β antagonist activity.

The present invention provides compounds which are selective $\beta_3$ receptor agonists. As such, the compounds effectively lead to an increase in insulin sensitivity and are useful in treating Type II diabetes and other ailments implicated by the $\beta_3$ receptor, without cardiac or tremor-related side effects.

**Summary of Invention**

The present invention provides compounds of the Formula I:

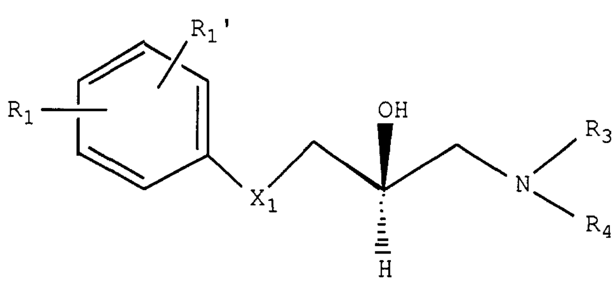

(I)

wherein:

$R_1$ is OH, halo, $SO_2NHR_2$, $CO_2R_2$, $CONHR_2$, $NHCOR_2$, -NH(optionally substituted aryl), $CF_3$, or $CF_2H$;
$R_1'$ is H, halo, $C_1$-$C_4$ alkyl, OH, $SO_2NHR_2$, $CO_2R_2$, $CONHR_2$, $NHCOR_2$, $CF_3$ or $CF_2H$;
$R_2$ is H, $C_1$-$C_4$ alkyl, or aryl;
$R_3$ is H or $C_1$-$C_4$ alkyl;
$R_4$ is a moiety selected from the group consisting of:

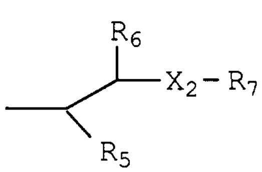

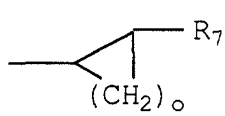

or

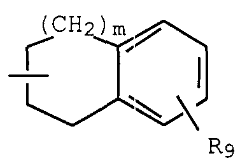

;

$R_5$ and $R_6$ are independently $C_1$-$C_4$ alkyl;
$R_7$ is an optionally substituted heterocycle or

a group selected from the group consisting of:

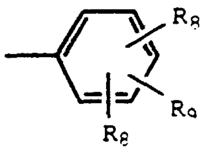
,
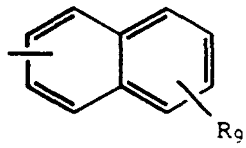
,
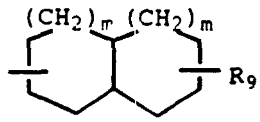
,

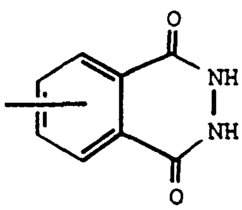
,
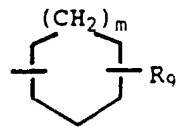
,
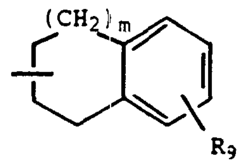
;

$R_8$ is independently H, halo or $C_1$-$C_4$ alkyl;
$R_9$ is halo, CN, $OR_{10}$, $C_1$-$C_4$ alkyl, $C_1$-$C_4$ haloalkyl, $CO_2R_2$, $CONR_{11}R_{12}$, CONH($C_1$-$C_4$ alkyl or $C_1$-$C_4$ alkoxy), $SR_2$, $CSNR_2$, $CSNR_{11}R_{12}$, $SO_2R_2$, $SO_2NR_{11}R_{12}$, $SOR_2$, $NR_{11}R_{12}$, aryl, heterocycle, optionally substituted aryl, optionally substituted heterocycle, or $C_1$-$C_4$ alkyl or $C_2$-$C_4$ alkenyl optionally substituted with CN;
$R_{10}$ is independently $C_1$-$C_4$ alkyl, $C_1$-$C_4$ haloalkyl, $(CH_2)_nC_3$-$C_8$ cycloalkyl, $(CH_2)_n$aryl, $(CH_2)_n$heterocycle, $(CH_2)_n$ optionally substituted $C_3$-$C_8$ cycloalkyl, $(CH_2)_n$ optionally substituted aryl, or $(CH_2)_n$ optionally substituted heterocycle;
$R_{11}$ and $R_{12}$ are independently H, $C_1$-$C_4$ alkyl, or combine with the nitrogen to which each are bound to form morpholinyl, piperidinyl, pyrrolidinyl, or piperazinyl;
$X_1$ is O or S;
$X_2$ is absent or a 1 to 5 carbon straight or branched alkylene;
m is 0 or 1;
n is 0, 1, 2, or 3;
o is 1, 2, 3, 4, 5, or 6;

or a pharmaceutically acceptable salt or solvate thereof.

The present invention also provides a novel process for making compounds of Formula I.

The compounds of the present invention are selective $\beta_3$ receptor agonists and as such are useful for treating Type II diabetes and obesity, as well as useful for stimulating the $\beta_3$ receptor. Therefore, the present invention also provides for methods of treating Type II diabetes and obesity, as well as a method of stimulating the $\beta_3$ receptor.

In addition, the present invention provides the use of compounds of Formula I for treating Type II diabetes and obesity as well the use of compounds of Formula I for stimulating the $\beta_3$ receptor.

## Detailed Description

For the purposes of the present invention, as disclosed and claimed herein, the following terms are defined below. As they relate to the present invention, the terms below may not be interpreted, individually or collectively, to describe chemical structures that are unstable or impossible to construct.

The term "halo" represents fluorine, chlorine, bromine, or iodine.

The term "$C_1$-$C_4$ alkyl" represents a cyclo, straight or branched chain alkyl group having from one to four carbon atoms such as methyl, ethyl, n-propyl, isopropyl, cyclopropyl, n-butyl, isobutyl, sec-butyl, t-butyl and the like. A "haloalkyl" is one such alkyl substituted with one or more halo atoms, preferably one to three halo atoms. An example of a haloalkyl is trifluoromethyl. An "alkoxy" is a alkyl group covalently bonded by an -O- linkage.

The term "1 to 5 carbon straight or branched alkylene" represents a one to five carbon, straight or branched,

alkylene moiety. A branched alkylene may have one or more points of branching. A 1 to 5 carbon straight or branched alkylene may optionally be unsaturated at one or more carbons. Thus, a 1 to 5 carbon straight or branched alkylene includes 1 to 5 carbon alkylene, alkenylene and alkylidene moieties. Examples include methylene, ethylene, propylene, butylene, -CH(CH3)CH2-CH($C_2H_5$)$CH_2$-, -CH($CH_3$)CH($CH_3$)-, -$CH_2$C($CH_3$)$_2$-, -$CH_2$CH($CH_3$)$CH_2$-, -C($CH_3$)$_2$CH=, -CH=CH$CH_2$-, -CH=CH-, and the like.

The "acyl" moiety, alone or in combination, is derived from an alkanoic acid containing from one to seven carbon atoms. The term "acyl" also includes moieties derived from an aryl carboxylic acid.

The term "aryl" represents an optionally substituted or unsubstituted phenyl or naphthyl. The term $(CH_2)_n$aryl is preferably benzyl or phenyl.

The term "optionally substituted" as used herein means an optional substitution of one to three, preferably one or two groups independently selected from halo, $C_1$-$C_4$ haloalkyl, hydroxy, carboxy, tetrazolyl, acyl, $COOR_2$, $CONR_{11}R_{12}$, CONH($C_1$-$C_4$ alkoxy), cyano, $C_1$-$C_4$ alkoxy, $C_1$-$C_4$ alkyl, phenyl, benzyl, nitro, $NR_{11}R_{12}$, NHCO($C_1$-$C_4$ alkyl), NHCO(benzyl), NHCO(phenyl), $SR_2$, S($C_1$-$C_4$ alkyl), OCO($C_1$-$C_4$ alkyl), $SO_2$($NR_{11}R_{12}$), $SO_2$($C_1$-$C_4$ alkyl), or $SO_2$(phenyl); provided that such substitution does not entirely destroy biological activity, as defined in this specification.

$R_{11}$ and $R_{12}$ are independently H, $C_1$-$C_4$ alkyl, or combine with the nitrogen to which each is bound to form morpholinyl, piperidinyl, pyrrolidinyl, or piperazinyl.

The term "heterocycle" represents a stable, optionally substituted or unsubstituted, saturated or unsaturated 5 or 6 membered ring, said ring having from one to four heteroatoms that are the same or different and that are selected from the group consisting of sulfur, oxygen, and nitrogen; and when heterocycle contains two adjacent carbon atoms, the adjacent carbon atoms may be structured to form a group of the formula -CH=CH-; provided that (1) when the heterocyclic ring contains 5 members, the heteroatoms comprise not more than two sulfur or two oxygen atoms but not both; and (2) when the heterocyclic ring contains 6 members and is aromatic, sulfur and oxygen are not present. The heterocycle may be attached at any carbon or nitrogen which affords a stable structure. The heterocycle may be optionally substituted. Examples of an heterocycle include pyrazole, pyrazoline, imidazole, isoxazole, triazole, tetrazole, oxazole, 1,3-dioxolone, thiazole, oxadiazole, thiadiazole, pyridine, pyrimidine, piperazine, morpholine, pyrazine, pyrrolidine, piperidine, oxazolidone, oxazolidinedione, imidazolidinone.

The term "leaving group" as used in the specification is understood by those skilled in the art. Generally, a leaving group is any group or atom that enhances the electrophilicity of the atom to which it is attached for displacement. Preferred leaving groups are p-nitrobenzene sulfonate, triflate, mesylate, tosylate, imidate, chloride, bromide, and iodide.

The term "pharmaceutically effective amount", as used herein, represents an amount of a compound of the invention that is capable of stimulating the $\beta_3$ receptor in mammals. The particular dose of the compound administered according to this invention will, of course, be determined by the particular circumstances surrounding the patient, including the compound administered, the route of administration, the particular condition being treated, and similar considerations.

The term "unit dosage form" refers to physically discrete units suitable as unitary dosages for human subjects and other mammals, each unit containing a predetermined quantity of active material calculated to produce the desired therapeutic effect, in association with a suitable pharmaceutical carrier.

The term "treating," as used herein, describes the management and care of a patient for the purpose of combating the disease, condition, or disorder and includes the administration of a compound of present invention to prevent the onset of the symptoms or complications, to alleviate symptoms or complications, or to eliminate the disease, condition, or disorder.

The term "selective" means preferential agonism of the $\beta_3$ receptor over agonism of the $\beta_1$ or $\beta_2$ receptor. In general, the compounds of the present invention demonstrate at a minimum a twenty fold differential (preferably over a 50x differential) in the dosage required to behave as an agonist to the $\beta_3$ receptor and the dosage required for equal agonism of the $\beta_1$ and $\beta_2$ as measured in the Functional Agonist Assay. The compounds demonstrate this differential across the range of doses. Thus, $\beta_3$ selective compounds behave as agonists for the $\beta_3$ receptor at much lower concentrations with lower toxicity by virtue of their minimal agonism of the other receptors.

As previously noted, the present invention provides compounds of the Formula I.

Preferred compounds are those of Formula Ia

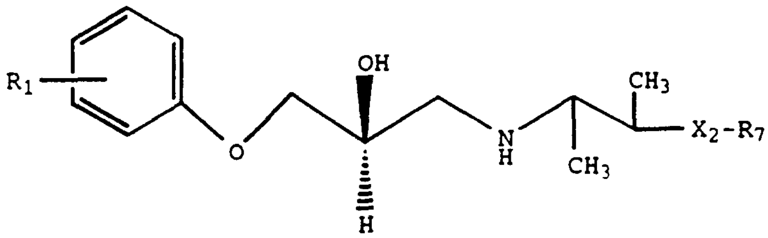

(Ia)

wherein:

$R_1$ is F, OH, $-SO_2NR_2$, $-NHCOR_2$, or $CF_2H$;
$R_7$ is

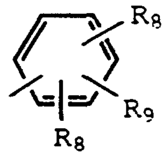

or

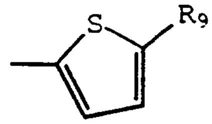

;

$R_8$ is independently H, halo or $C_1$-$C_4$ alkyl;
$R_9$ is $OR_{10}$, tetrazolyl, $CONR_{11}R_{12}$, or $SO_2NR_{11}R_{12}$;
$R_{10}$ is $(CH_2)_n$aryl, $(CH_2)_n$heterocycle, said aryl or heterocycle being optionally substituted with tetrazolyl, CN, $CO_2R_2$, $CONR_{11}R_{12}$, or $SO_2NR_{11}R_{12}$;
$R_{11}$ and $R_{12}$ are independently H, $C_1$-$C_4$ alkyl, or combine with the nitrogen to which each is bound to form morpholinyl, piperidinyl, pyrrolidinyl or piperazinyl;
$X_2$ is 1 to 2 carbon alkylene;
n is 0, 1, 2, or 3;

or a pharmaceutically acceptable salt.

Other preferred compounds are those of Formula Ib:

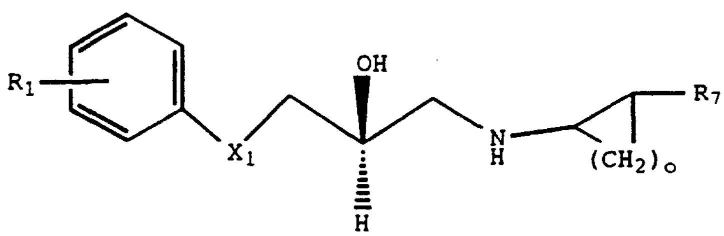

(Ib)

wherein:

$R_1$ is F, $-SO_2NR_2$, $-NHCOR_2$, or $CF_2H$;
$R_7$ is an optionally substituted heterocycle or

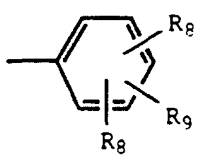

;

$R_8$ is independently H, halo or $C_1$-$C_4$ alkyl;
$R_9$ is $OR_{10}$, $CONR_{11}R_{12}$, or $SO_2NR_{11}R_{12}$;
$R_{10}$ is independently $(CH_2)_n$aryl, $(CH_2)_n$heterocycle, said aryl or heterocycle being optionally substituted with tetrazolyl, CN, $CO_2R_2$, $CONR_{11}R_{12}$, or $SO_2NR_{11}R_{12}$;

$R_{11}$ and $R_{12}$ are independently H, $C_1$-$C_4$ alkyl, or combine with the nitrogen to which each is bound to form morpholinyl, piperdinyl, pyrrolidinyl or piperazinyl;
$X_1$ is O or S;
n is 0, 1, 2, or 3;
o is 1, 2, 3, 4, 5, or 6;

or a pharmaceutically acceptable salt or solvate thereof.

Additional preferred compounds are those of the Formula Ic:

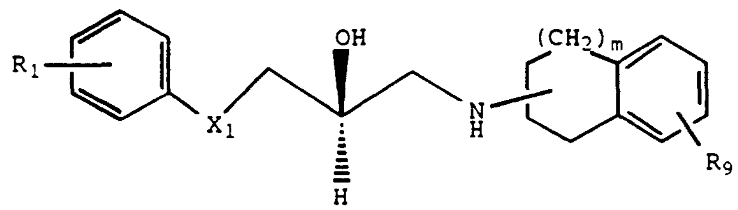

(Ic)

wherein:

$R_1$ is F, $-SO_2NR_2$, $-NHCOR_2$, or $CF_2H$;
$R_9$ is $OR_{10}$, $CONR_{11}R_{12}$, $SO_2NR_{11}R_{12}$, $SOR_2$, $NR_{11}R_{12}$, optionally substituted aryl, optionally substituted aryloxy, or optionally substituted heterocycle;
$R_{10}$ is $(CH_2)_n$aryl, $(CH_2)_n$heterocycle, said aryl or heterocycle being optionally substituted with tetrazolyl, $CONR_{11}R_{12}$, or $SO_2NR_{11}R_{12}$;
$R_{11}$ and $R_{12}$ are independently H, $C_1$-$C_4$ alkyl, or combine with the nitrogen to which each is bound to form morpholinyl, piperidinyl, pyrrolidinyl, or piperazinyl;
$X_1$ is O or S;
m is 0 or 1;
n is 0, 1, 2, or 3;

or a pharmaceutically acceptable salt or solvate thereof.

Particularly preferred compounds are those of Formula Ia, Ib or Ic, wherein $R_9$ is $CONH_2$ and $OR_{10}$, wherein $R_{10}$ is a optionally substituted aryl, particularly phenyl, or optionally substutituted heterocycle, particularly pyridine.

Additional preferred compounds include the following:

(All isomers of:) 5-{3-[2-hydroxy-3-(2-fluorophenyloxy)-propylamino]-2-methylbutyl)-thiophene-2-sulfonamide

(All isomers of:) 5-(2-fluoro-4-{3-[2-hydroxy-3-(2-fluorophenyloxy)-propylamino] -2-methylbutyl}-phenyl )-1H-tetrazole

(All isomers of:) 4-{3-[2-hydroxy-3-(3-hydroxyphenyloxy)propylamino]-2-methylbutyl)benzamide

(All isomers of:) 4-{3-[2-hydroxy-3-(2-fluorophenyloxy)propylamino]-2-methylbutyl}-3-methylbenzamide

(All isomers of:) 4-{3-[2-hydroxy-3-(3-acetylaminophenyloxy)-propylamino]-2-methylbutyl}benzamide

By virtue of their acidic moieties, some of the compounds of Formula I include the pharmaceutically acceptable base addition salts thereof. Such salts include those derived from inorganic bases such as ammonium and alkali and alkaline earth metal hydroxides, carbonates, bicarbonates, and the like, as well as salts derived from basic organic amines such as aliphatic and aromatic amines, aliphatic diamines, hydroxy alkamines, and the like. Such bases useful in preparing the salts of this invention thus include ammonium hydroxide, potassium carbonate, sodium bicarbonate, calcium hydroxide, methylamine, diethylamine, ethylenediamine, cyclohexylamine, ethanolamine and the like.

Because of the basic moiety, some of the compounds of Formula I can also exist as pharmaceutically acceptable acid addition salts. Acids commonly employed to form such salts include inorganic acids such as hydrochloric, hydrobromic, hydroiodic, sulfuric and phosphoric acid, as well as organic acids such as paratoluenesulfonic, methanesulfonic, oxalic, parabromophenylsulfonic, carbonic, succinic, citric, benzoic, acetic acid, and related inorganic and organic acids. Such pharmaceutically acceptable salts thus include sulfate, pyrosulfate, bisulfate, sulfite, bisulfite, phosphate, mono-hydrogenphosphate, dihydrogenphosphate, metaphosphate, pyrophosphate, chloride, bromide, iodide,

acetate, propionate, decanoate, caprylate, acrylate, formate, isobutyrate, heptanoate, propiolate, oxalate, malonate, succinate, suberate, sebacate, fumarate, maleate, 2-butyne-1,4 dioate, 3-hexyne-2, 5-dioate, benzoate, chlorobenzoate, hydroxybenzoate, methoxybenzoate, phthalate, xylenesulfonate, phenylacetate, phenylpropionate, phenylbutyrate, citrate, lactate, hippurate, β-hydroxybutyrate, glycollate, maleate, tartrate, methanesulfonate, propanesulfonate, naphthalene-1-sulfonate, naphtalene-2-sulfonate, mandelate and the like salts.

It is recognized that various stereoisomic forms of the compounds of Formulas I may exist. The compounds may be prepared as racemates and can be conveniently used as such. Therefore, the racemates, individual enantiomers, diastereomers, or mixtures thereof form part of the present invention. Unless otherwise specified, whenever a compound is described or referenced in this specification all the racemates, individual enantiomers, diastereomers, or mixtures thereof are included in said reference or description.

The compounds of Formula I are prepared as described in the following Schemes and examples.

## Scheme I

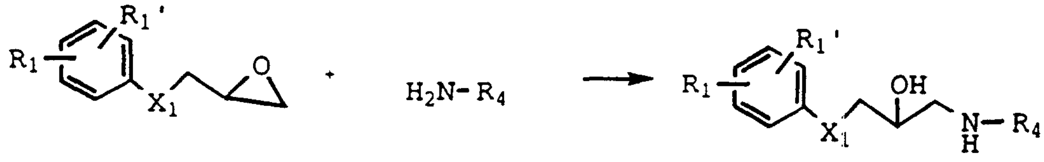

(II) (III)

In Scheme I, $R_1$, $R_1$' and $R_4$ have the same meaning as previously described. The reaction of Scheme I is carried out under conditions appreciated in the art for the amination of epoxides. For example, the epoxide (II) may be combined with the amine (III) in an alcohol, preferably, ethanol at room temperature to the reflux temperature of the reaction mixture. Preferably, the reaction is carried out under conditions generally described in Atkins et al., Tetrahedron Lett. 27:2451 (1986). These conditions include mixing the reagents in the presence of trimethylsilylacetamide in a polar aprotic solvent such as acetonitrile, dimethylformamide (DMF), acetone, dimethylsulfoxide (DMSO), dioxane, diethylene glycol methyl ether (diglyme), tetrahydrofuran (THF), or other polar aprotic solvents in which the reagents are soluble. Preferably, the solvent is DMSO. The reaction is carried out at temperatures ranging from about 0°C to reflux.

The compounds of the present invention can be prepared by a novel combinatorial/parallel synthesis. This synthesis is described in Scheme II.

## Scheme II

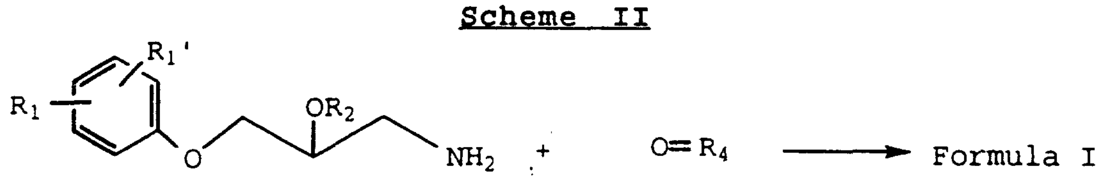


(IV) (V)

In Scheme II, $R_1$, $R_1$', $R_2$, and $R_4$ have the same meaning as previously described. The reaction of Scheme II is preferably carried out by adding to a glass vial: a non-reactive solvent such as methanol, DMF, methylene chloride or acetonitrile, amine (IV), and ketone (V). The solution is shaken to allow for imine formation and treated with Amberlite IRA400 borohydride resin (Aldrich). The slurry is then shaken an additional 24 hours to effect reduction to the secondary amine. Methylene chloride and polystyrene-linked benzaldehyde resin (Frechet, J.M. et al., J. Am Chem. Soc. 93:492 (1971)) is added to the vial, in order to scavenge excess primary amine starting material. The slurry is shaken, preferably overnight. The slurry is then filtered through a cotton plug, and the residual solids rinsed with methanol. Evaporation under a flow of air, followed by drying for several hours at room temperature in a vacuum oven yields the desired product of sufficient purity.

A modification of Scheme II is necessary when the amine hydrochloride salt is used. Addition of resin-bound base to the initial reaction mixture prior to reduction or scavenging allows the desired reaction to proceed. Imine formation using amine hydrochloride salts, an aldehyde or ketone, and a resin bound amine base may be carried out using two different resins: poly(4-vinylpyridine), commercially available from Aldrich, and resin (VIII), synthesized by

the reaction of Merrifield resin with piperidine (Scheme IIa):

**Scheme IIa**

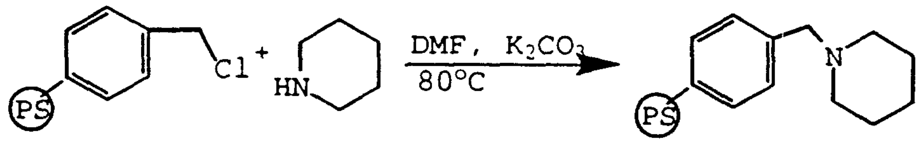


(VI) (VII) (VIII)

In Scheme IIa, PS is polysytrene. Both the poly(4-vinylpyridine) and resin (VIII) promote imine formation.

Scheme II can also be carried out by utilization of traditional techniques. Reductive aminations described in scheme II are well known in the art. They are typically performed by mixing the amine and ketone starting materials in a solvent and adding a reducing agent. Solvents typically include lower alcohols, DMF, and the like. A wide variety of reducing agents can be utilized, most commonly utilized are sodium borohydride and sodium cyanoborohydride. The reaction is typically performed at room temperature to the reflux temperature of the solvent. Products are isolated by techniques well known in the art.

Many of the ketone and amino starting materials utilized in Scheme II can be prepared by techniques recognized and appreciated to one skilled in the art. The synthesis of additional starting materials is generally described in Schemes III and IV. **Scheme III**

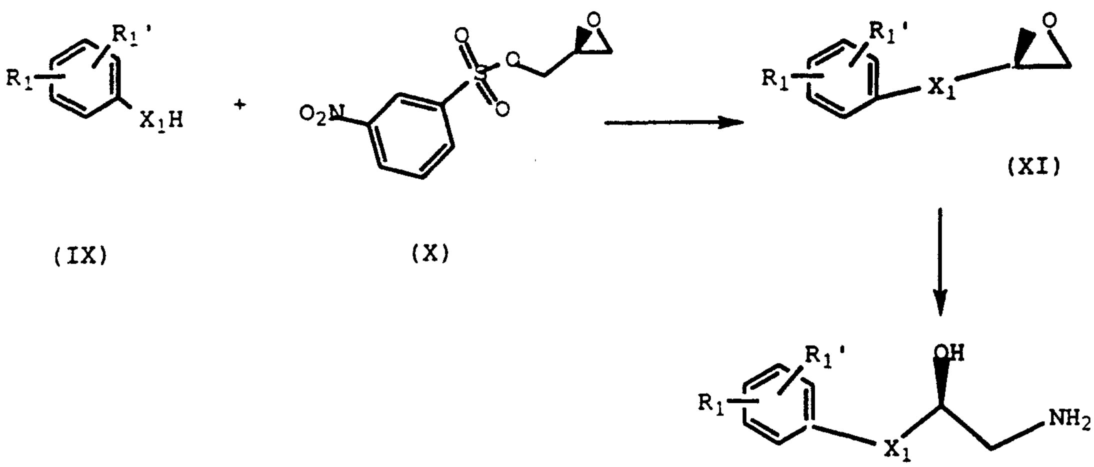

(IX) (X) (XI) (XII)

In Scheme III, $X_1$, $R_1$' and $R_1$ are the same as previously defined. Equimolar amounts of the aromatic compound (Compound IX) and (2S)-(+)-glycidyl 3-nitrobenzenesulfonate (Compound X) are dissolved in an inert solvent such as acetone and treated with 1.1 equivalents of a non-reactive acid scavenger, such as $K_2CO_3$. The suspension is then heated at reflux for 16-20 hours with stirring. The solvent is removed *in vacuo*. The residue is partitioned between chloroform or other organic solvent and water. The organic layer is dried over $Na_2SO_4$ and concentrated *in vacuo* to give the epoxide (XI) in sufficient purity (>95%) and yield (85-100%).

The epoxide (XI) is dissolved in an alcohol, preferably methanol, and treated with one equivalent of dibenzylamine. The solution is preferably stirred at reflux for three to four hours and then cooled to ambient temperature. Approximately 10 equivalents of ammonium formate are added to the flask, followed by 10% palladium on carbon, and the suspension stirred vigorously at reflux for 30-45 minutes. The reaction mixture is then filtered through Celite, concentrated *in vacuo* to a minimum volume and treated with 1.1 equivalents of a 1.0 M anhydrous solution of HCl in ether. The solution is concentrated to dryness. The solid residue is triturated with pentane to yield products of sufficient purity (>97%) and yield (60-100%). If desired, further purification may be carried out by passing over a short plug of silica, eluting with $CHCl_3$, then 95:5 $CHCl_3$/MeOH, then 25:5:1 $CHCl_3$/MeOH/$NH_4OH$.

Alternatively, the epoxide (XI) is treated with a solution of methanol saturated with ammonia gas and stirred at room temperature in a sealed tube for 16 hours. This solution is then evaporated, and the residue subjected to standard purifications such as column chromatography or recrystallization. The HCl salt is then optionally produced by the addition of HCl gas in ether.

The reaction of Scheme III is further described in Beedle et al., U.S. patent 5,013,761 and reference cited therein. U.S. patent 5,013,761 is herein incorporated by reference.

The ketone moieties utilized in Scheme II that are either unknown in the art or not commercially available are prepared in accordance with Scheme IV.

Scheme IV

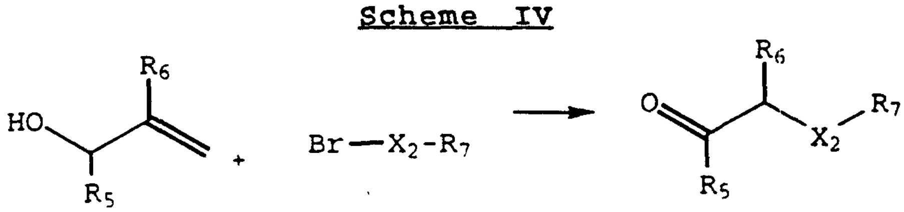

(XIII) (XIV) (XV)

In Scheme IV, $X_2$, $R_4$, $R_5$, $R_6$, and $R_7$ are the same as previously defined. Preferably, $R_4$ is a substituted phenyl. The reaction described in Scheme IV is referred to as a Heck reaction and is described in A.J. Chalk et al., J. Org. Chem. 41: 1206 (1976). The reaction is achieved by treating compound (XIII) with an arylpalladium reagent. The arylpalladium reagent is generated in situ by treating Compound (XIV) with a palladium-triarylphosphine complex. The reaction is generally carried out in under conditions appreciated in the art.

Another embodiment of the current invention is a process of preparing a compound of Formula I which comprises:

In step 1, reacting an epoxide of the formula:

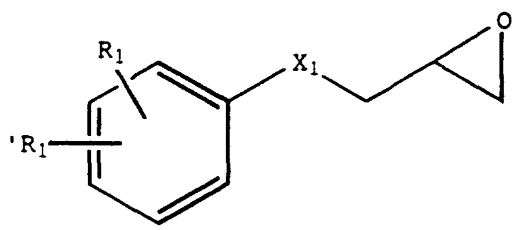

(II)

with an amine of formula (B):

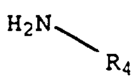

(III);

and in step 2, reacting the product of step 1 to form an acid addition salt.

Starting materials for the compounds described in Schemes I, II, III, and IV are either commercially available, known in the art, or can be prepared by methods known in the art or described herein.

The following examples and preparations are provided merely to further illustrate the invention. The scope of the invention is not construed as merely consisting of the following examples. In the following examples and preparations, melting point, nuclear magnetic resonance spectra, mass spectra, high pressure liquid chromatography over silica gel, gas chromatography, N,N-dimethylformamide, palladium on charcoal, tetrahydrofuran, ethyl acetate, thin layer chromatography and elemental analysis are abbreviated M.Pt., NMR, MS, HPLC, GC, DMF, Pd/C, THF, EtOAc, TLC and EA respectively. The terms "EA", "NMR", and "MS" indicate that the data was consistent with the desired structure.

Preparation 1

(S)-3-(2-fluorophenyl)-1,2-epoxypropane

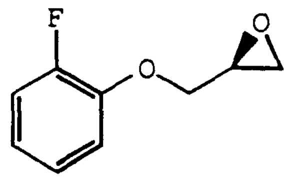

A solution of 2-fluorophenol (865 mg, 7.72mol) and (2S)-(+)-glycidyl-3-nitrobenzenesulfonate (2.0 g, 7.72 mmol) in 50 mL of acetone was treated with 1.1 equivalents of $K_2CO_3$ (1.17 g, 8.5 mmol) and stirred at reflux for 18 hours. The suspension was cooled to ambient temperature, the solids filtered, and the filtrate concentrated *in vacuo* to dryness. The resulting solids were partitioned between chloroform and water, and the aqueous layer extracted once with chloroform. The organic layers were combined and dried over $Na_2SO_4$ and concentrated *in vacuo* to a colorless oil which slowly crystallized at 0°C to 1.11 g (86%) white needles. TLC (Rf = 0.5, $CHCl_3$) and NMR indicated >95% purity, so the material was used without further purification.

Preparation 2

(S)-3-(2-fluorophenyloxy)-2-hydroxypropy lammonium chloride

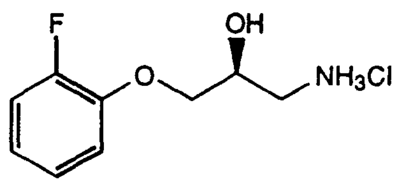

S-3-(2-fluorophenyl)-1,2-epoxypropane (1.08 g, 6.4 mmol) was dissolved in 50 mL of methanol and treated with dibenzylamine (1.23 mL, 6.4 mmol, d=1.026). The mixture was stirred at reflux for 3 hours and then cooled to ambient temperature. A vast excess of ammonium formate (3.0 g, 47.6 mmol) was added followed by 10% palladium on carbon (350 mg), and the suspension was stirred at reflux for 45 minutes. After cooling the suspension, the reaction mixture was filtered through Celite and the filtrate concentrated *in vacuo* to a colorless oil. The oil was redissolved in 10 mL of methanol and treated with a 1.0 M anhydrous solution of HCl in ether (7.0 mL, 7 mmol) and reduced *in vacuo* to dryness. The residue was triturated in pentane and the solids filtered to yield 1.4 g (99%) of a dry white powder. NMR. EA.

Preparation 3

(S) -3-(2-fluorophenylthio)-2-hydroxypropylamine

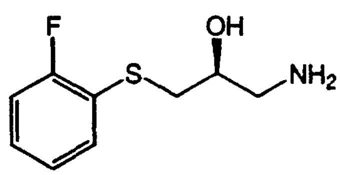

(2S)-(+)-glycidyl-3-nitrobenzenesulfonate (3.0 g, 11.6 mmol) and potassium carbonate (1.8 g, 13 mmol) in acetone (50 mL) was sparged with nitrogen for 5 minutes. 2-fluorothiophenol (1.5 g, 11.6 mmol) was added and the mixture stirred at ambient temperature under a blanket of nitrogen for 4 hours. The acetone was removed *in vacuo* and the residue partitioned between water/ethyl acetate. The organic layer was dried ($MgSO_4$) and concentrated to give 1.6 g (75%) of the intermediate epoxide as a colorless oil and was used without further purification.

The intermediate epoxide (1.2 g, 6.5 mmol) was dissolved in methanol (10 mL) and cooled to 0°C using an ice bath. The solution was saturated with ammonia gas and the reaction vessel was sealed and allowed to stir at ambient temperature for 16 hours. The reaction was opened at 0°C and the ammonia allowed to evaporate before the mixture was concentrated *in vacuo.* The residue was purified by flash chromatography on silica gel using 25:5:1 $CHCl_3$:$MeOH$:$NH_4OH$ to give 980 mg of a colorless oil (75%) which rapidly crystallized under vacuum to give a white solid, mp 50-53 °C. NMR. MS. EA.

<u>Preparation 4</u>

4-[(2-oxocyclohexyl)methyl]benzonitrile

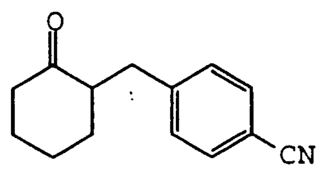

A mixture of methyl cyclohexanone-2-carboxylate (11.0 g, 70 mmol, from Fluka), α-bromo-p-tolunitrile (12.3 g, 63 mmol), potassium carbonate (10.5 g, 76 mmol) in THF (200 mL) was refluxed for 24 hours. The progress of the reaction was followed by GC. The reaction was diluted with water and the THF was removed under reduced pressure. The aqueous portion was extracted with EtOAc, dried ($MgSO_4$) to give 19.3 g of a white solid that was 74% pure by gas chromatrophy. The solid was recrystallized from hexane/EtOAc to give 7.75 g white crystals that were 100% pure by glc. A second crop of 3.65 g was obtained by adding more hexane to the filtrate. Overall, 11.4 g (67%) of 1-[(4-cyanophenyl)methyl]-1-methoxycarbonyl-2-oxocyclohexane carboxylate, was obtained; mp 82-84°C. NMR. MS.

Under a blanket of nitrogen, a mixture of 1-[(4-cyanophenyl) methyl]-1-methoxycarbonyl-2-oxocyclohexane carboxylate (7.6 g, 28 mmol), sodium cyanide (2.1 g, 42 mmol) and DMSO (100 mL) was heated at 115°C for 1.5 hours. The progress of the reaction was monitored by glc. The reaction was cooled and partitioned between water, EtOAc and brine. The organic layer was washed with water and dried ($MgSO_4$). After concentration, crude product was obtained as a tan oil. Purification by plug filtration (200 g silica gel, 15% EtOAc/hexane) gave 3.3 g (55%) product as colorless oil. NMR. MS.

<u>Preparation 5</u>

4-[(2-oxocyclohexyl)methyl]benzamide

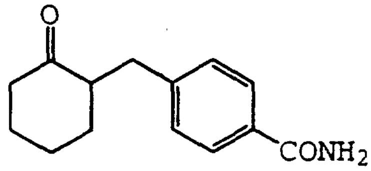

A DMSO (20 mL) solution of the compound of Preparation 28 (2.5 g, 11.7 mmol) was cooled in an ice bath. Solid $K_2CO_3$ (500 mg) was added followed immediately by 30% $H_2O_2$ (3 mL). After 20 minutes, TLC (3/7 EtOAc/hexane) showed a trace of starting material remained. The ice bath was removed and the reaction was stirred and room temperature for 1 hour. The reaction was diluted with 500 mL water and the white solid collected and dried to give 2.44 g (90%) desired amide. The product was recrystallized from 1/9 EtOAc/hexane to give 2.02 g of the titled

product as white crystals, mp 167-170°C. NMR. MS.

Preparation 6

2-Tetralone-6-carboxylic acid, ethylene ketal

6-bromo-2-tetralone (2.0 g, 8.89 mmol) was dissolved in toluene (50 mL) and treated with excess ethylene glycol (4.88 mL, 88.9 mmol) and catalytic p-toluenesulfonic acid (15 mg). The solution was stirred at reflux 16 hours, and water was removed from the reaction mixture using a Dean-Stark condenser. After cooling to ambient temperature, the toluene solution was washed 2 x 1N NaOH, 1 x water, 1 x brine, dried over $Na_2SO_4$ and concentrated *in vacuo* to give 2.23 g (93%) of 6-bromo-2-tetralone ethylene ketal as a brown oil which was used without further purification.

6-bromo-2-tetralone ethylene ketal (2.2 g, 8.15 mmol) was dissolved in anhydrous THF (30 mL), cooled to -78°C and treated with *tert*-butyllithium (12.05 mL, 20.4 mmol, 1.7M in pentane) under an atmosphere of nitrogen. After stirring for 30 minutes, anhydrous carbon dioxide gas was passed through the reaction mixture for 20 minutes at -78°C. The suspension was then allowed to warm to ambient temperature. The solution was quenched with water and acidified with 1N HCl, then extracted 2 x EtOAc. The organic extracts were washed with brine, dried over $Na_2SO_4$ and concentrated *in vacuo* to a pale brown oil. The oily residue was applied to a silica flash chromatography column and eluted with 30%-50% EtOAC in hexanes to yield 2-tetralone-6-carboxylic acid, ethylene ketal 1.06 g (55%) a slowly crystallizing solid. NMR. MS.

**Preparation 7**

2-Tetralone-6-carboxamide

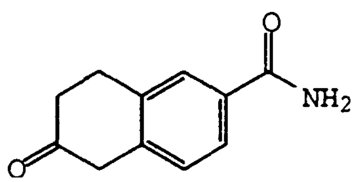

2-tetralone-6-carboxylic acid, ethylene ketal (395 mg, 2.07 mmol) was co-dissolved in $CH_2Cl_2$ (50 mL) with N-hydroxysuccinimide (260 mg, 2.76 mmol) at 0°C and treated with a slight excess of 1,3-dicyclohexylcarbodiimide (502 mg, 2.50 mmol). The mixture was allowed to warm to ambient temperature over 30 minutes, during which time a fine white precipitate formed. Ammonium chloride (333 mg 6.23 mmol) and triethylamine (1.58 mL, 12.5 mmol, d=0.797) were added and the solution stirred at ambient temperature for 16 hours. The suspended urea and salts were filtered away and the solution concentrated *in vacuo* to a colorless oil. The oil was applied to a silica flash chromatography column and eluted with 50-100% EtOAc in hexanes to yield 250 mg (64%) of 2-tetralone-6-carboxamide, ethylene ketal as a white solid, clean by NMR, TLC.

2-tetralone-6-carboxamide, ethylene ketal (250 mg, 1.07 mmol) and catalytic *p*-toluenesulfonic acid were stirred in acetone (50 mL) at ambient temperature for 48 hours. The volatiles were removed *in vacuo* and the residue triturated in ethyl acetate. The solids were filtered, washed and dried to yield 77.5 mg (38%) of 2-Tetralone-6-carboxamide as a white powder, pure by NMR, TLC. MS.

Preparation 8

2-Tetralone-6-morpholinamide

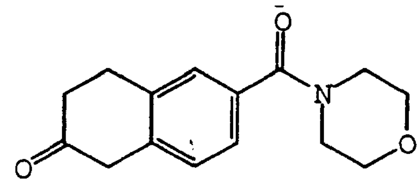

2-tetralone-6-carboxylic acid, ethylene ketal (395 mg, 2.07 mmol) was codissolved in CH2C12 (50 mL) with N-hydroxysuccinimide (260 mg, 2.76 mmol) at 0°C and treated with a slight excess of 1,3-dicyclohexylcarbodiimide (502 mg, 2.50 mmol). The mixture was allowed to warm to ambient temperature over 30 minutes, during which time a fine white precipitate formed. Morpholine (0.91 mL, 10.4 mmol, d=0.998) was added and the solution stirred at ambient temperature for 16 hours. The suspended urea was filtered away and the solution concentrated *in vacuo* to a colorless oil. The oil was applied to a silica flash chromatography column and eluted with 50-100% EtOAc in hexanes to yield 323 mg (51%) of 2-Tetralone-6-morpholinamide, ethylene ketal as a slowly crystallizing solid, clean by NMR, TLC.

2-Tetralone-6-morpholinamide, ethylene ketal (323 mg, 1.06 mmol) and catalytic *p*-toluenesulfonic acid were stirred in acetone (50 mL) at ambient temperature for 48 hours. TLC indicated a mixture of 2-tetralone-6-morpholinamide, ethylene ketal and desired product, so the solution was heated to reflux for 16 hours. The volatiles were removed *in vacuo* and the residue applied to a silica flash chromatography column and eluted with 50-100% EtOAc in hexanes to yield 27 mg (10%) of 2-tetralone-6-morpholinamide a slowly crystallizing solid, pure by NMR, TLC. MS.

Preparation 9

5-[2-fluoro-4 -(2-methyl-3-oxobutyl)-phenyl]-tetrazole

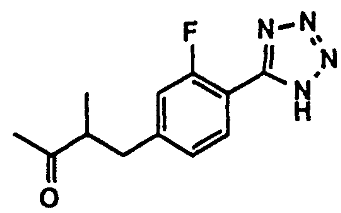

4-bromo-2-fluorobenzonitrile (5.3 g, 26.5 mmol) and 3-methyl-3-buten-2-ol (3.5 g, 40 mmol) were dissolved in N-methylpyrrolidinone (30 mL) and treated with catalytic palladium diacetate (115 mg, 0.5 mmol), tris-(o-tolyl)-phosphine (300 mg, 1.0 mmol), and $NaHCO_3$ (2.7 g, 32 mmol). The mixture was stirred at 120°C for one hour. The solution was cooled to ambient temperature and partitioned between $H_2O$/ethyl acetate, dried ($MgSO_4$) and concentrated *in vacuo*. The residue was applied to a silica chromatography column and eluted with 4:1 hexane/ethyl acetate to yield 2.3 g of a pale yellow oil (43%)

The oil (1.3 g, 6.3 mmol) was dissolved in DMF (30 mL) and treated with sodium azide (455 mg, 7 mmol) and ammonium chloride (375 mg, 7 mmol) and stirred at 90°C for 16 hours. The reaction mixture was concentrated *in vacuo* and partitioned between 3N NaOH/diethyl ether. The aqueous layer was acidified with conc. HCl and cooled, and then extracted with diethyl ether, dried ($MgSO_4$), and concentrated *in vacuo* to a pale brown oil. The residue was applied to a silica chromatography column and eluted with 25:5:1 $CHCl_3$/MeOH/$NH_4$OH to yield 140 mg of a white solid (9%).

Preparation 10

5-(3 -methyl-3-oxobutyl)-thiophene-2-sulfonamide

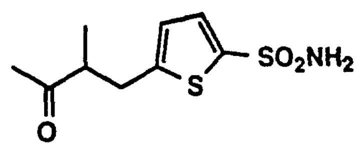

5-bromothiophene-2-sulfonamide (5.2 g, 21.5 mmol) and 3-methyl-3-buten-2-ol (2.8 g, 32.2 mmol) were dissolved in N-methylpyrrolidinone (40 mL) and treated with catalytic palladium diacetate (96 mg, 0.43 mmol), tris-(o-tolyl)-phosphine (262 mg, 0.86 mmol), and $NaHCO_3$ (2.2 g, 25.8 mmol). The mixture was stirred at 160'C for 48 hours. TLC indicated the reaction was ca. 50% completed at that time. The solution was cooled to ambient temperature and partitioned between $H_2O$/ethyl acetate, dried (MgSO4) and concentrated *in vacuo* to a dark brown oil. The residue was applied to a silica chromatography column and eluted with 2:3 hexane/ethyl acetate to yield 220 mg of a pale brown oil (4.1%).

Preparation 11

4-(2-methyl-3-oxobutyl)benzamide

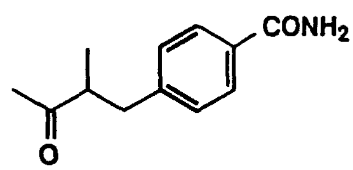

4-bromobenzonitrile (9.1 g, 50 mmol) and 3-methyl-3-buten-2-ol (6.5 g, 75 mmol) were dissolved in N-methylpyrrolidinone (40 mL) and treated with catalytic palladium diacetate (225 mg, 1.0 mmol), tris-(o-tolyl)-phosphine (610 mg, 2.0 mmol), and $NaHCO_3$ (5.0 g, 60 mmol). The mixture was stirred at 120°C for three hours. The solution was cooled to ambient temperature and partitioned between $H_2O$/ethyl acetate, dried ($MgSO_4$) and concentrated *in vacuo*. The residue was applied to a silica chromatography column and eluted with 3:1 hexane/ethyl acetate to yield 6.2 g of a pale yellow oil (66%)

The oil (4.6 g, 24.6 mmol) was dissolved in DMSO (20 mL) and treated with $K_2CO_3$ (1.0 g) and $H_2O_2$ (6 mL, 30% w/w) and stirred at 0°C for 10 minutes. The reaction mixture was diluted with 500 mL water and saturated with NaCl, and then extracted with ethyl acetate. The organic extracts were dried ($MgSO_4$), and concentrated *in vacuo* to a white solid. The solid was recrystallized from 1:1 ethyl acetate/hexane to yield 3.22 g of a white solid (64%). M.P. 112-115.

The following compounds were prepared in a manner analogous to the schemes and/or preparations described herein or by techniques appreciated in the art:

| Name | Structure | m.p. | Yield | Confirmed by NMR | Confirmed by M.S. |
|---|---|---|---|---|---|
| (4-(1-methyl-3-oxobutyl)phenyl) methanenitrile<br><br>Preparation 12 | 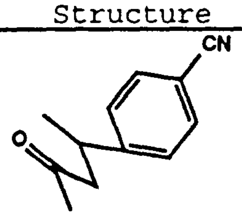 | oil | 44% | x | x |
| 4-(1-methyl-3-oxobutyl)benzamide<br><br>Preparation 13 | 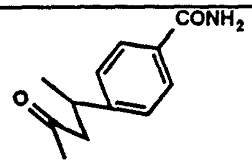 | 127-9 | 95% | x | x |
| 4-(3-oxocyclohexyl) benzonitrile<br><br>Preparation 14 | 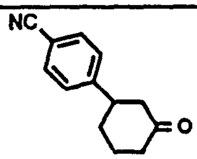 | 66-69 | 36% | x | x |
| 4-(2-methyl-3-oxobutyl)benzonitrile<br><br>Preparation 15 | 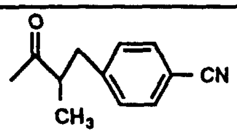 | oil | 66% | x | x |

| | | | | | |
|---|---|---|---|---|---|
| 4-(1,2-dimethyl-3-oxobutyl)benzamide<br><br>Preparation 16 | 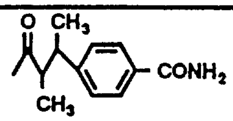 | 100-102 | 90% | x | x |
| 4-(3-oxocyclohexyl) benzamide<br><br>Preparation 17 | 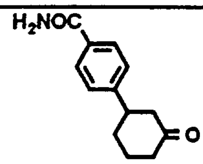 | 188-91 | 42% | x | x |
| 4-(3-oxocyclopentyl) benzamide<br><br>Preparation 18 | 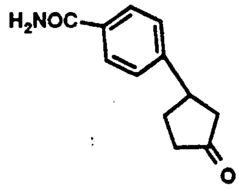 | 203-4 | 43% | x | x |
| 4-(1,1-dimethyl-3-oxobutyl)benzamide<br><br>Preparation 19 | 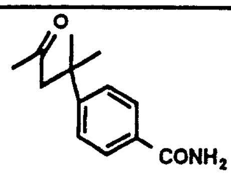 | 106-8 | 61% | x | x |
| 4-(2-oxocycloheptylmethyl) benzonitrile<br><br>Preparation 20 | 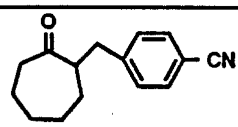 | oil | 54% | x | x |
| 4-(2,2-dimethyl-3-oxobutyl)benzonitrile<br><br>Preparation 21 | 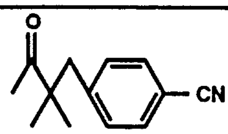 | oil | 72% | x | x |
| 4-(2,2-dimethyl-3-oxobutyl)benzamide<br><br>Preparation 22 | 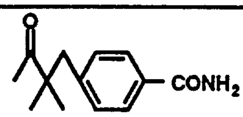 | 127-131 | 62% | x | x |
| 5-(2-methyl-3-oxobutyl)-2-thiophene sulfonamide<br><br>Preparation 23 | 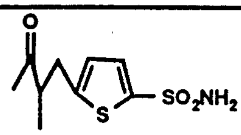 | oil | low | x | x |
| 4-((2-oxocycloheptyl) methyl)benzamide<br><br>Preparation 24 | 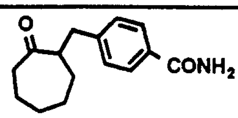 | 132-4 | 88% | x | x |

| | | | | | |
|---|---|---|---|---|---|
| 4-((2-oxocyclopentyl) methyl)benzonitrile<br><br>Preparation 25 | 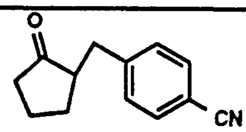 | oil | 62% | x | x |
| 4-((2-oxocyclopentyl) methyl)benzamide<br><br>Preparation 26 | 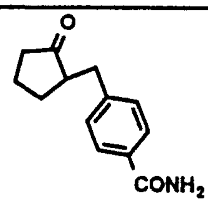 | 138-142 | 81% | x | x |
| 4-((2-oxocyclohexyl) methyl)benzonitrile<br><br>Preparation 27 | 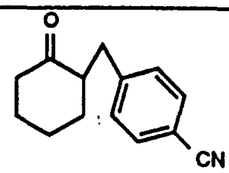 | oil | 55% | x | x |
| 4-((2-oxocyclohexyl) methyl)benzamide<br><br>Preparation 28 | 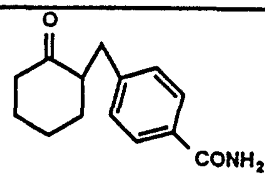 | 167-70 | 90% | x | x |
| 2-fluoro-4-(2-methyl-3-oxobutyl)benzonitrile<br><br>Preparation 29 | 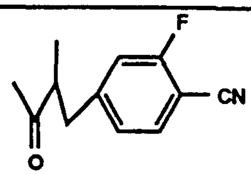 | oil | 42% | x | x |
| 2-fluoro-4-(2-methyl-3-oxobutyl)benzamide<br><br>Preparation 30 | 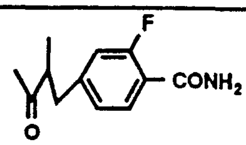 | 112-15 | 93% | x | x |
| 5-(2-methyl-3-oxobutyl)-2-(morpholinosulfonyl)-thiophene<br>Preparation 31 | 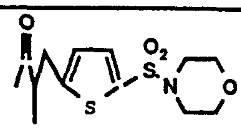 | oil | 15% | x | x |
| 3-methyl-4-(2-methyl-3-oxobutyl)benzonitrile<br><br>Preparation 32 | 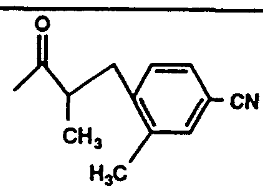 | oil | 64% | x | x |

| | | | | | |
|---|---|---|---|---|---|
| (3-methyl-4-(2-methyl-3-oxobutyl)benzamide<br><br>Preparation 33 | 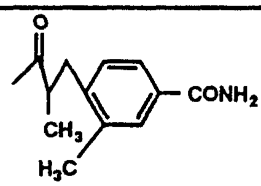 | 105-7 | 71% | x | x |
| 4-(2-ethyl-3-oxobutyl)benzoic acid<br><br>Preparation 34 | 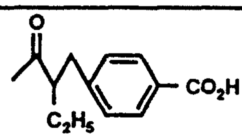 | | 24% | x | x |
| (2S)-3-amino-1-(3-methylphenoxy)-2-propanol<br><br>Preparation 35 | 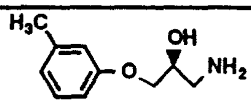 | 158-62 | low | x | x |
| (2S)-3-amino-1-(2-fluorothiophenyl)-2-propanol<br><br>Preparation 36 | 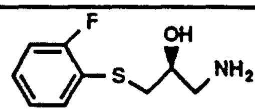 | 50-3 | 75% | x | x |
| (2S)-3-amino-1-(3-fluorothiophenol)-2-propanol<br><br>Preparation 37 | 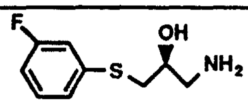 | 115-19 | 45% | x | x |
| (2S)-3-amino-1-(4-fluorothiophenol)-2-propanol<br><br>Preparation 38 | 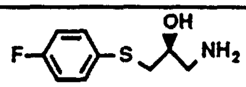 | 67-70 | 44% | x | x |
| (2S)-3-amino-1-(3-hydroxythiophenol)-2-propanol<br><br>Preparation 39 | 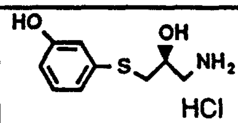 | oil | 15% | x | x |
| (2S)-3-amino-1-(3-difluoromethylphenoxy)-2-propanol<br><br>Preparation 40 | 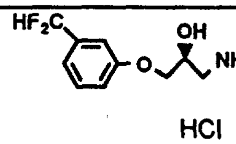 | 79-83 | 72% | x | x |
| (2S)-3-amino-2-(3-hydroxy-6-ethylphenoxy)-2-propanol<br><br>Preparation 41 | 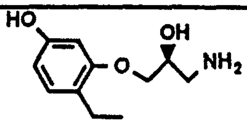 | hygro-scopic | 60% | x | |

Example 1

A 5x8 grid of 4 mL screw cap vials was arranged. To each of the eight rows of vials in the grid was added 33 μmol of ketone (from preparations 4-34, or commercially available), one ketone per row, as a stock solution in methanol (0.5M, 65 μl). If solubility was a problem, acetonitrile/methanol or DMF was used. To each column of vials in the grid

was added 50 μmol of amine hydrochloride, one amine hydrochloride (or amine) (from preparations 2, 3, 36-41 or commercially available) per column, as a stock solution in methanol (0.5M, 100 μl). To each vial was then added resin VIII (18-20 mg), 1.01 meq/g, 70-90μeq base). Teflon lined caps were then placed on each vial. The slurries were then shaken for 24 hours, at which time each vial was treated with approximately 30 mg (2.5 mmol $BH_4$-/g resin, 75 μmol) of Amberlite IRA400 borohydride resin (Aldrich Chemical). The caps were replaced, and the vials were shaken for an additional 24 hours, then 150 μl methylene chloride and 40 mg (1 mmol/g resin, 0.4 mmol) polystyrene-linked benzaldehyde resin (Frechet, J.M.; Schuerch, C.J. Am. Chem. Soc. 1971, 93, 492.) in order to scavenge excess primary amine starting material were added to the vial, and the slurry was shaken for 1 day. Each vial was then filtered through a cotton plug. The residual resin was rinsed with three small portions of methanol (approximately 200 μl). The resulting solutions were then treated with 20μl of conc. HCl (120 μmol) to ensure formation of the HCl salt of the product amine, then each vial was diluted to a volume of approximately 4 mL, and 1 mL of each solution was transferred to a tared 4 mL screw cap vial. This solution was allowed to evaporate in a fume hood under an air stream until dry, then placed in a vacuum oven for 24 hours at room temperature. The resulting residues were then weighed and submitted directly for testing with no further purification. The bulk of the material (75%) was similarly evaporated.

The following matrices list additional examples 2-81. These compounds were prepared using combinatorial/ parallel techniques in accordance with the present invention. All reaction conditions were the same from plate to plate and in substantial accordance with Scheme 2 and Example 1. The scaffold for each plate was the same and is depicted at the top corner of the 5x8 matrix. The variable functional groups are illustrated in the rows and columns. The ketones and the amines depicted on each plate were prepared in accordance with the schemes and preparations described herein or by techniques known in the art.

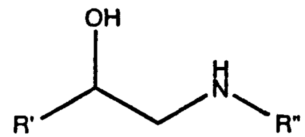

| R' ' = <br> R' = | 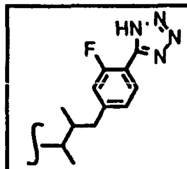 | 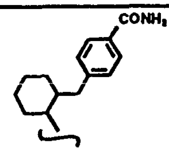 | 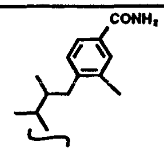 | 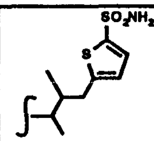 | 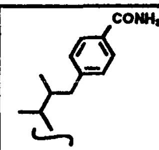 | 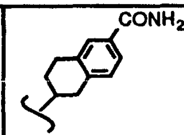 | 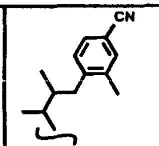 | 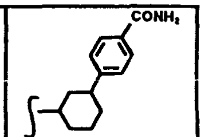 |
|---|---|---|---|---|---|---|---|---|
| 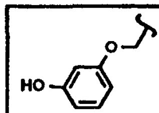 | Example 2 | Example 3 | Example 4 | Example 5 | Example 6 | Example 7 | Example 8 | Example 9 |
| 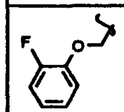 | Example 10 | Example 11 | Example 12 | Example 13 | Example 14 | Example 15 | Example 16 | Example 17 |
| 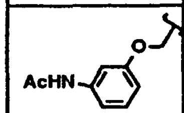 | Example 18 | Example 19 | Example 20 | Example 21 | Example 22 | Example 23 | Example 24 | Example 25 |
| 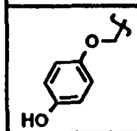 | Example 26 | Example 27 | Example 28 | Example 29 | Example 30 | Example 31 | Example 32 | Example 33 |
| 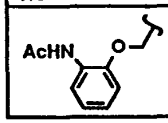 | Example 34 | Example 35 | Example 36 | Example 37 | Example 38 | Example 39 | Example 40 | Example 41 |

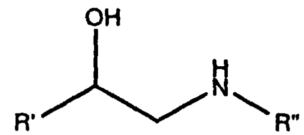

| R' = / R'' = | 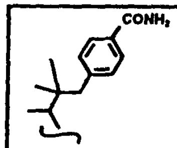 | 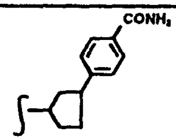 | 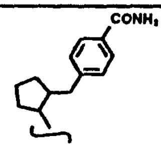 | 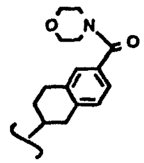 | 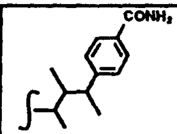 | 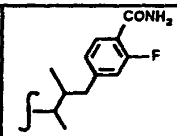 | 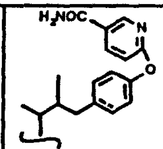 | 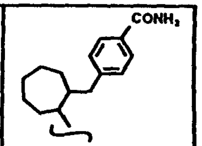 |
|---|---|---|---|---|---|---|---|---|
| 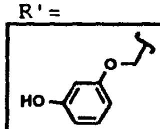 | Example 42 | Example 43 | Example 44 | Example 45 | Example 46 | Example 47 | Example 48 | Example 49 |
| 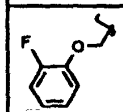 | Example 50 | Example 51 | Example 52 | Example 53 | Example 54 | Example 55 | Example 56 | Example 57 |
| 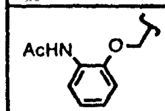 | Example 58 | Example 59 | Example 60 | Example 61 | Example 62 | Example 63 | Example 64 | Example 65 |
| 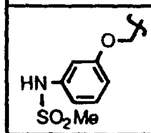 | Example 66 | Example 67 | Example 68 | Example 69 | Example 70 | Example 71 | Example 72 | Example 73 |
| 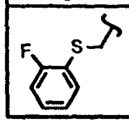 | Example 74 | Example 75 | Example 76 | Example 77 | Example 78 | Example 79 | Example 80 | Example 81 |

As previously noted, the compounds of the present invention are potent, selective β3 adrenergic receptor agonists. This pharmacological activity was determined in the functional agonist β3 assay.

Functional Agonists

$\beta_3$ Assay

Fell Lines

The h$\beta_2$ DNA was expressed from a plasmid 57537 obtained from American Type Culture Collection. h$\beta_1$ and h$\beta_3$ adrenergic receptors were cloned from human genomic libraries using the polymerase chain reaction method with degenerate probes. Full length receptors were cloned, expressed and sequenced to verify identity according to published sequences (h$\beta_{1:}$ T. Frielle et. al. (1993) Molecular Pharmacology 44: 264-270). These receptors were then expressed in the DXB-11 variant of CHO cells using a vector restoring tetrahydrofolate reductase and hygromycin resistance. Rat $\beta_3$ receptor expressing CHO cell line is known in the art. Mol. Pharm., Vol 40, pp. 895-99 (1991). CHO cells were grown in 10% dialyzed FBS./high glucose DMEM/0.1% proline.

cAMP Assay

Cell membranes were harvested from the above cell line using hypotonic 25 mM Hepes (pH 7.4), 1 mM EDTA, 20 µg/mL leupeptin, 1 mM PMSF buffer with scraping followed by differential centrifugation. Membranes were incubated in 25 mM Tris (pH 7.6), 0.2% BSA, 2.6 mM Mg, 0.8 mM ATP, 0.1 mM GTP, 5 mM creatine phosphate, creatine kinase 50 U/mL, 0.2 mM IBMX at 32°C. Agonists were added and incubation continued for 15 m. cAMP produced was assayed using a fluorescent tracer-immuno assay method.

Intact cell assays were performed using suspended cells removed from culture flasks by trypsin treatment. Cells were preincubated with 0.5 mM IBMX at 37°C. Agonists were added and incubation continued for 15 m. Incubation was stopped by heating suspension in boiling water. cAMP or cGMP in these and the soleus incubations were assayed by RIA (Amersham).

The compounds of the invention are agonists of the $\beta_3$ receptor. Isoproterenol is accepted in the art as a non-selective $\beta_3$ agonist and is widely used as a comparator in evaluating the activity of compounds. See Trends in Pharm. Sci. 15: 3 (1994). In the Functional Agonist $\beta_3$ assay, the compounds demonstrated at least 30%, preferably 50% and most preferably over 85% of isoproterenol's response at a single dose of 50µmol. Dose response titrations on the agonists described reveal $EC_{50}$ values of < 10 µM, preferably < 1mM. In the functional assay, dose titration furnishes an $EC_{50}$ for isoproterenol of 1.1±0.5 mM.

When screened against the $\beta_1$ and $\beta_2$ receptors in the functional assay, dose titration experiments indicate that greatly reduced or no receptor stimulation is observed with the compounds of the invention. This is defined by measuring the intrinsic activity (maximal response achieved) as compared to isoproterenol. The claimed compounds of Formula I are selective $\beta_3$ receptor agonists and have an intrinsic activity of < 3% of isoproterenol's response.

Thus, the compounds of the invention are selective $\beta_3$ adrenergic receptor agonists.

As agonists of $\beta_3$, the compounds are useful in treating conditions in a mammal in which the $\beta_3$ receptor has been demonstrated to play a role. The prefered mammal of treatment is a human. The relationship between modulating the $\beta_3$ receptor and treatment of diseases, such Type II diabetes and obesity, is well established in the art. Other conditions recognized in the art include: asthma, depression, and gastrointestinal disorders such as gastrointestinal motility. Thus, the present compounds are useful in the treatment of inflammatory bowel disease (Crohn's disease or ulcerative colitis), irritable bowel syndrome, non-specific diarrhoea dumping syndrome, asthma, and depression.

In treating non-human mammals, the compounds of the present invention are useful for increasing weight gain and/or improving the feed utilization efficiency and/or increasing lean body mass and/or decreasing birth mortality rate and increasing post/natal survival rate.

The compounds of Formula I are preferably formulated prior to administration. Therefore, yet another embodiment of the present invention is a pharmaceutical formulation comprising a compound of Formula I and one or more pharmaceutically acceptable carriers, diluents or excipients.

The present pharmaceutical formulations are prepared by known procedures using well-known and readily available ingredients. In making the compositions of the present invention, the active ingredient will usually be mixed with a carrier, or diluted by a carrier, or enclosed within a carrier which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders.

Some examples of suitable carriers, excipients, and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient.

The compositions are preferably formulated in a unit dosage form, each dosage containing from about 0.1 to about 500 mg, preferably about 5 to about 200 mg, of the active ingredient. However, it will be understood that the therapeutic dosage administered will be determined by the physician in the light of the relevant circumstances including the condition to be treated, the choice of compound to be administered and the chosen route of administration, and therefore, the above dosage ranges are not intended to limit the scope of the invention in any way. The compounds can be administered by a variety of routes including the oral, rectal, transdermal, subcutaneous, topical, intravenous, intramuscular or intranasal routes. For all indications, a typical daily dose will contain from about 0.05 mg/kg to about 20 mg/kg of the active compound of this invention. Preferred daily doses will be about 0.1 to about 10 mg/kg, ideally about 0.1 to about 5 mg/kg. However, for topical administration a typical dosage is about 1 to about 500 µg compound per $cm^2$ of an affected tissue. Preferably, the applied amount of compound will range from about 30 to about 300 µg/$cm^2$, more preferably, from about 50 to about 200 µg/$cm^2$, and, most preferably, from about 60 to about 100 µg/$cm^2$.

The following formulation example is illustrative only and is not intended to limit the scope of the invention in any way.

Formulation 1

Hard gelatin capsules are prepared using the following ingredients:

| | Quantity (mg/capsule) |
|---|---|
| 4-{3-[2-hydroxy-3-(3-hydroxyphenyloxy)-propylamino]-2-methylbutyl}benzamide | 25 |
| starch, dried | 425 |
| magnesium stearate | 10 |
| Total | 460 mg |

The above ingredients are mixed and filled into hard gelatin capsules in 460 mg quantities.

The principles, preferred embodiments and modes of operation of the present invention have been described in the foregoing specification. The invention which is intended to be protected herein, however, is not to be construed as limited to the particular forms disclosed, since they are to be regarded as illustrative rather than restrictive. Variations and changes may be made by those skilled in the art without departing from the spirit of the invention.

## Claims

1. A compound of Formula:

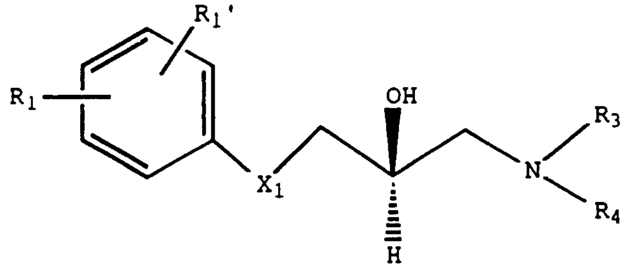

(I)

wherein:

$R_1$ is OH, halo, $SO_2NHR_2$, $CO_2R_2$, $CONHR_2$, $NHCOR_2$, -NH(optionally substituted aryl), $CF_3$, or $CF_2H$;

$R_1$' is H, halo, $C_1$-$C_4$ alkyl, OH, $SO_2NHR_2$, $CO_2R_2$, $CONHR_2$, $NHCOR_2$, $CF_3$ or $CF_2H$;
$R_2$ is H, $C_1$-$C_4$ alkyl, or aryl;
$R_3$ is H or $C_1$-$C_4$ alkyl;
$R_4$ is a moiety selected from the group consisting of:

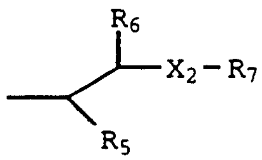
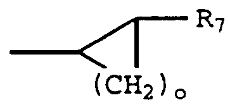
or
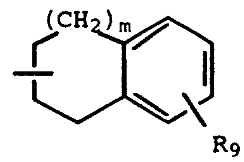
;

$R_5$ and $R_6$ are independently $C_1$-$C_4$ alkyl;
$R_7$ is an optionally substituted heterocycle or a group selected from the group consisting of:

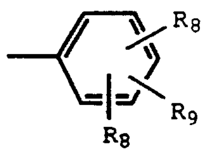
,
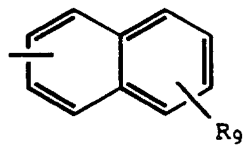
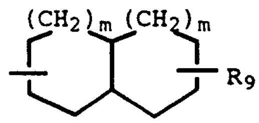
,

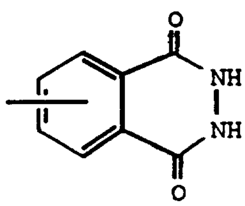
,
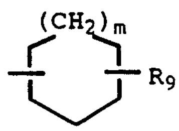
,
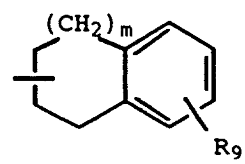
;

$R_8$ is independently H, halo or $C_1$-$C_4$ alkyl;
$R_9$ is halo, CN, $OR_{10}$, $C_1$-$C_4$ alkyl, $C_1$-$C_4$ haloalkyl, $CO_2R_2$, $CONR_{11}R_{12}$, CONH($C_1$-$C_4$ alkyl or $C_1$-$C_4$ alkoxy), $SR_2$, $CSNR_2$, $CSNR_{11}R_{12}$, $SO_2R_2$, $SO_2NR_{11}R_{12}$, $SOR_2$, $NR_{11}R_{12}$, aryl, heterocycle, optionally substituted aryl, optionally substituted heterocycle, or $C_1$-$C_4$ alkyl or $C_2$-$C_4$ alkenyl optionally substituted with CN;
$R_{10}$ is independently $C_1$-$C_4$ alkyl, $C_1$-$C_4$ haloalkyl, $(CH_2)_nC_3$-$C_8$ cycloalkyl, $(CH_2)_n$aryl, $(CH_2)_n$heterocycle, $(CH_2)_n$ optionally substituted $C_3$-$C_8$ cycloalkyl, $(CH_2)_n$ optionally substituted aryl, or $(CH_2)_n$ optionally substituted heterocycle;
$R_{11}$ and $R_{12}$ are independently H, $C_1$-$C_4$ alkyl, or combine with the nitrogen to which each are bound to form morpholinyl, piperidinyl, pyrrolidinyl, or piperazinyl;
$X_1$ is O or S;
$X_2$ is absent or a 1 to 5 carbon straight or branched alkylene;
m is 0 or 1;
n is 0, 1, 2, or 3;
o is 1, 2, 3, 4, 5, or 6;

or a pharmaceutically acceptable salt or solvate thereof.

**2.** A compound of Claim 1 wherein $R_1$ is halo, $CF_3$, $CONHR_2$, or NH(optionally substituted aryl); and
$R_4$ is

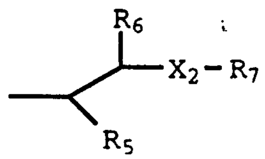, or 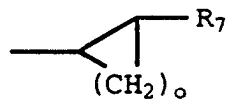.

**3.** A compound of Claim 2 wherein $R_7$ is

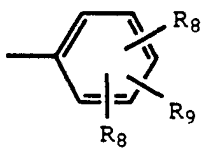, 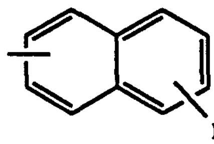, or 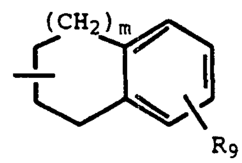; and

$R_9$ is halo, CN, $OR_{10}$, $CO_2R_2$, $CONR_{11}R_{12}$, $SR_2$, $SO_2R_2$, $SO_2NR_{11}R_{12}$, $SOR_2$ or $NR_{11}R_{12}$.

**4.** A compound of Claim 3 wherein $R_7$ is

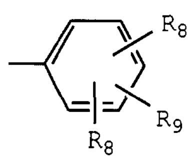.

and
$R_9$ is halo, CN, $CO_2R_2$, or $OR_{10}$.

**5.** A compound of any of Claims 1 through 4 for use as a pharmaceutical.

**6.** A compound as claimed in Claim 5 for use in treating Type II diabetes.

**7.** A compound as claimed in Claim 5 for use in treating obesity.

**8.** A compound as claimed in Claim 5 for use in agonizing the $\beta_3$ receptor.

**9.** A pharmaceutical formulation comprising as an active ingredient a compound of any one of Claims 1 through 4, associated with one or more pharmaceutically acceptable carriers, excipients or diluents.

**10.** A process of preparing a compound of Formula I which comprises:
In step 1, reacting an epoxide of the formula:

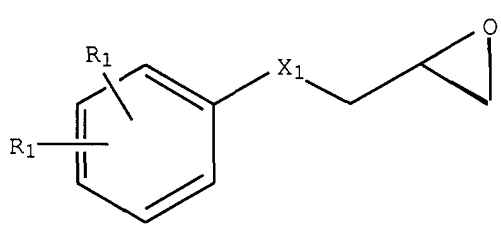

(II)

with an amine of formula (B):

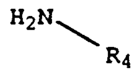 (III);

and in step 2, reacting the product of step 1 to form an acid addition salt.

**Patentansprüche**

**1.** Verbindung der Formel

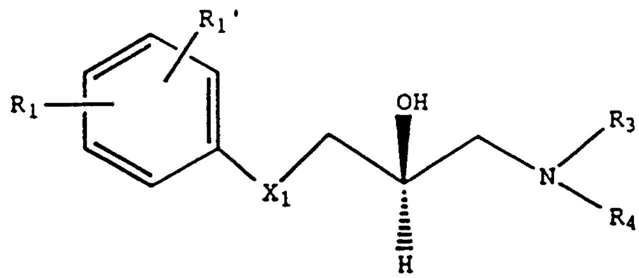

(I)

worin

$R_1$ für OH, Halogen, $SO_2NHR_2$, $CO_2R_2$, $CONHR_2$, $NHCOR_2$, -NH-(wahlweise substituiertes Aryl), $CF_3$ oder $CF_2H$ steht,
$R_1$' für H, Halogen, $C_1$-$C_4$ Alkyl, OH, $SO_2NHR_2$, $CO_2R_2$, $CONHR_2$, $NHCOR_2$, $CF_3$ oder $CF_2H$ steht,
$R_2$ für H, $C_1$-$C_4$ Alkyl oder Aryl steht,
$R_3$ für H oder $C_1$-$C_4$ Alkyl steht,
$R_4$ für einen Rest steht, der aus der Gruppe ausgewählt ist, die besteht aus

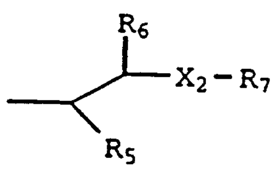

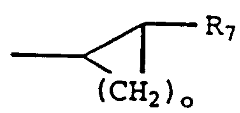

oder

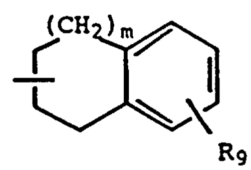

$R_5$ und $R_6$ unabhängig für $C_1$-$C_4$ Alkyl stehen,
$R_7$ für einen wahlweise substituierten Heterocyclus oder eine Gruppe steht, die aus der Gruppe ausgewählt ist, welche besteht aus

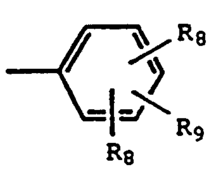

,

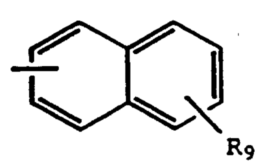

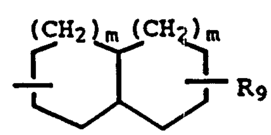

,

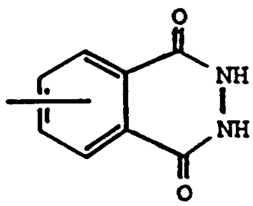 , 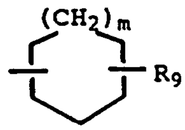 , 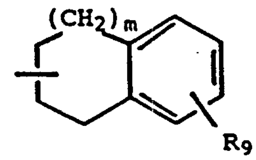

$R_8$ unabhängig für H, Halogen oder $C_1$-$C_4$ Alkyl steht,
$R_9$ steht für Halogen, CN, $OR_{10}$, $C_1$-$C_4$ Alkyl, $C_1$-$C_4$ Halogenalkyl, $CO_2R_2$, $CONR_{11}R_{12}$, CONH($C_1$-$C_4$ Alkyl oder $C_1$-$C_4$ Alkoxy), $SR_2$, $CSNR_2$, $CSNR_{11}R_{12}$, $SO_2R_2$, $SO_2NR_{11}R_{12}$, $SOR_2$, $NR_{11}R_{12}$, Aryl, Heterocyclus, wahlweise substituiertes Aryl, wahlweise substituierter Heterocyclus oder $C_1$-$C_4$ Alkyl oder $C_2$-$C_4$ Alkenyl, das wahlweise mit CN substituiert ist,
$R_{10}$ unabhängig für $C_1$-$C_4$ Alkyl, $C_1$-$C_4$ Halogenalkyl, $(CH_2)_nC_3$-$C_8$ Cycloalkyl, $(CH_2)_n$Aryl, $(CH_2)_n$Heterocyclus, $(CH_2)_n$-wahlweise substituiertes $C_3$-$C_8$ Cycloalkyl, $(CH_2)_n$-wahlweise substituiertes Aryl oder $(CH_2)_n$-wahlweise substituierter Heterocyclus steht,
$R_{11}$ und $R_{12}$ unabhängig für H oder $C_1$-$C_4$ Alkyl stehen oder mit dem Stickstoff an den sie jeweils gebunden sind, unter Bildung von Morpholinyl, Piperidinyl, Pyrrolidinyl oder Piperazinyl kombinieren,
$X_1$ für O oder S steht,
$X_2$ fehlt oder für ein gerades oder verzweigtes Alkylen mit 1 bis 5 Kohlenstoffen steht,
m für 0 oder 1 steht,
n für 0, 1, 2 oder 3 steht,
o für 1, 2, 3, 4, 5 oder 6 steht
oder ein pharmazeutisch annehmbares Salz oder Solvat hiervon.

**2.** Verbindung nach Anspruch 1, worin $R_1$ für Halogen, $CF_3$, $CONHR_2$ oder NH(wahlweise substituiertes Aryl) steht und $R_4$ steht für

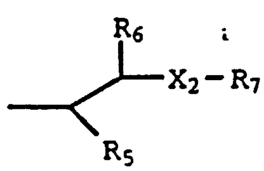 oder 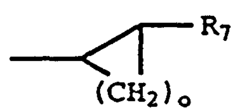

**3.** Verbindung nach Anspruch 2, worin $R_7$ steht für

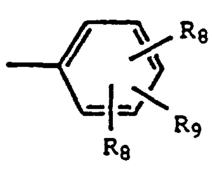 , 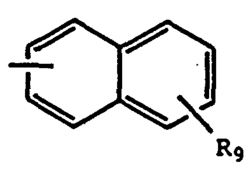 , oder 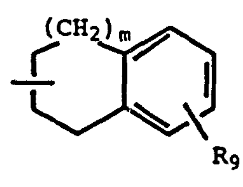

und
$R_9$ für Halogen, CN, $OR_{10}$, $CO_2R_2$, $COR_{11}R_{12}$, $SR_2$, $SO_2R_2$, $SO_2NR_{11}R_{12}$, $SOR_2$ oder $NR_{11}R_{12}$ steht.

**4.** Verbindung nach Anspruch 3, worin $R_7$ steht für

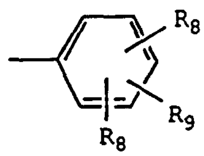

und

$R_9$ für Halogen, CN, $CO_2R_2$ oder $OR_{10}$ steht.

**5.** Verbindung nach einem der Ansprüche 1 bis 4 zur Verwendung als Pharmazeutikum.

**6.** Verbindung nach Anspruch 5 zur Verwendung bei der Behandlung von Typ II Diabetes.

**7.** Verbindung nach Anspruch 5 zur Verwendung bei der Behandlung von Fettsucht.

**8.** Verbindung nach Anspruch 5 zur Verwendung bei der Agonistenwirkung am $\beta_3$-Rezeptor.

**9.** Pharmazeutische Formulierung, die als Wirkstoff eine Verbindung nach einem der Ansprüche 1 bis 4 zusammen mit einem oder mehreren pharmazeutisch annehmbaren Trägern, Hilfsstoffen oder Verdünnungsmitteln enthält.

**10.** Verfahren zur Herstellung einer Verbindung der Formel I, gekennzeichnet durch in Schritt 1 Umsetzung eines Epoxids der Formel

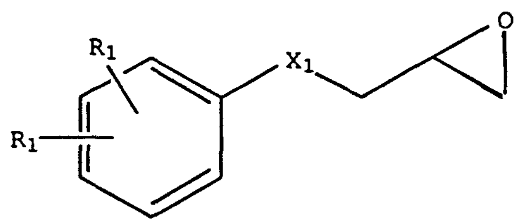

(II)

mit einem Amin der Formel (B)

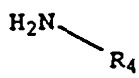

(III)

und in Schritt 2 Umsetzung des Produkts von Schritt 1 unter Bildung eines Säureadditionssalzes.

**Revendications**

**1.** Composé répondant à la Formule :

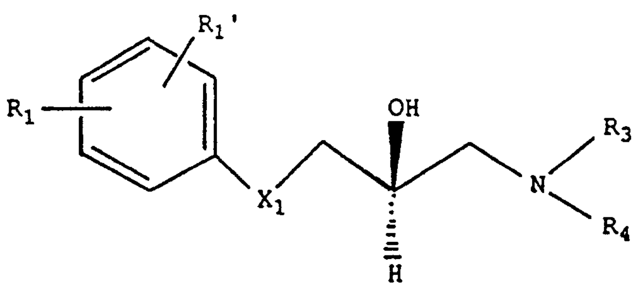

(I)

dans laquelle :

$R_1$ représente un groupe OH, un atome d'halogène, un groupe $SO_2NHR_2$, un groupe $CO_2R_2$, un groupe $CONHR_2$, un groupe $NHCOR_2$, un groupe -NH(aryle facultativement substitué), un groupe $CF_3$, ou un groupe $CF_2H$;
$R_1$' représente un atome d'hydrogène, un atome d'halogène, un groupe alkyle en $C_1$-$C_4$, un groupe OH, un groupe $SO_2NHR_2$, un groupe $CO_2R_2$, un groupe $CONHR_2$, un groupe $NHCOR_2$, un groupe $CF_3$ ou un groupe $CF_2H$ ;
$R_2$ représente un atome d'hydrogène, un groupe alkyle en $C_1$-$C_4$ ou un groupe aryle ;
$R_3$ représente un atome d'hydrogène ou un groupe alkyle en $C_1$-$C_4$;
$R_4$ représente une fraction choisie dans le groupe constitué de :

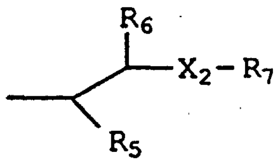
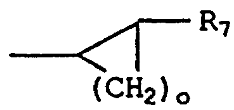 ou 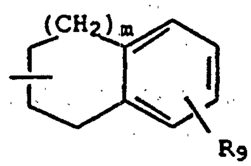 ;

$R_5$ et $R_6$ représentent indépendamment un groupe alkyle en $C_1$-$C_4$;
$R_7$ représente un hétérocycle facultativement substitué ou un groupe choisi dans le groupe constitué de :

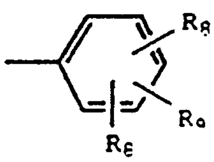 , 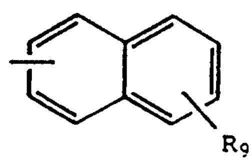 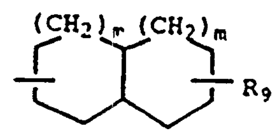 ,

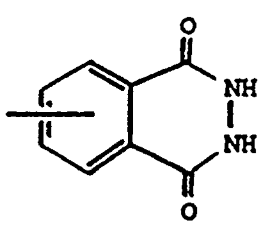 , 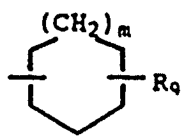 , 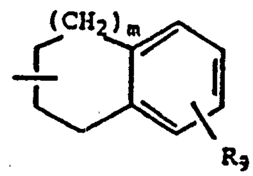 ;

$R_8$ représente indépendamment un atome d'hydrogène, un atome d'halogène ou un groupe alkyle en $C_1$-$C_4$;
$R_9$ représente un atome d'halogène, un groupe CN, un groupe $OR_{10}$, un groupe alkyle en $C_1$-$C_4$, un groupe halogénoalkyle en $C_1$-$C_4$, un groupe $CO_2R_2$, un groupe $CONR_{11}R_{12}$, un groupe CONH(alkyle en $C_1$-$C_4$ ou alcoxy en $C_1$-$C_4$), un groupe $SR_2$, un groupe $CSNR_2$, un groupe $CSNR_{11}R_{12}$, un groupe $SO_2R_2$, un groupe $SO_2NR_{11}R_{12}$, un groupe $SOR_2$, un groupe $NR_{11}R_{12}$, un groupe aryle, un hétérocycle, un groupe aryle facultativement substitué, un hétérocycle facultativement substitué, ou un groupe alkyle en $C_1$-$C_4$ ou alcényle en $C_2$-$C_4$ facultativement substitué par un groupe CN ;
$R_{10}$ représente indépendamment un groupe alkyle en $C_1$-$C_4$, un groupe halogénoalkyle en $C_1$-$C_4$, un groupe $(CH_2)_n$(cycloalkyle en $C_3$-$C_8$), un groupe $(CH_2)_n$aryle, un groupe $(CH_2)_n$hétérocycle, un groupe $(CH_2)_n$(cycloalkyle en $C_3$-$C_8$ facultativement substitué), un groupe $(CH_2)_n$(aryle facultativement substitué), ou un groupe $(CH_2)_n$(hétérocycle facultativement substitué) ;
$R_{11}$ et $R_{12}$ représentent indépendamment un atome d'hydrogène, un groupe alkyle en $C_1$-$C_4$, ou se combinent avec l'atome d'azote auquel ils sont liés chacun, pour former un groupe morpholinyle, un groupe pipéridinyle, un groupe pyrrolidinyle ou un groupe pipérazinyle ;
$X_1$ représente un atome O ou un atome S ;
$X_2$ est absent ou représente un groupe alkylène linéaire ou ramifié avec 1 à 5 atomes de carbone ;
m vaut 0 ou 1 ;

n vaut 0, 1, 2 ou 3 ;
o vaut 1, 2, 3, 4, 5 ou 6 ;

ou un sel ou solvate pharmaceutiquement acceptable de celui-ci.

2. Composé selon la revendication 1, dans lequel $R_1$ représente un atome d'halogène, un groupe $CF_3$, un groupe $CONHR_2$ ou un groupe -NH(aryle facultativement substitué) ; et
$R_4$ représente

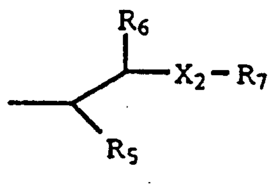 , ou 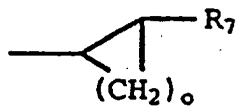 .

3. Composé selon la revendication 2, dans lequel $R_7$ représente

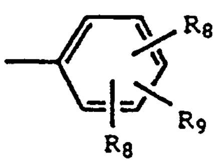 , 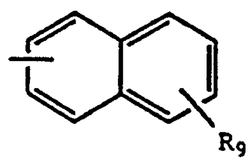 , ou 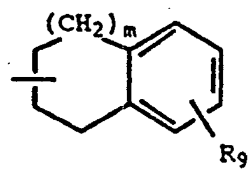 ; et

$R_9$ représente un atome d'halogène, un groupe CN, un groupe $OR_{10}$, un groupe $CO_2R_2$, un groupe $CONR_{11}R_{12}$, un groupe $SR_2$, un groupe $SO_2R_2$, un groupe $SO_2NR_{11}R_{12}$, un groupe $SOR_2$ ou un groupe $NR_{11}R_{12}$.

4. Composé selon la revendication 3, dans lequel $R_7$ représente

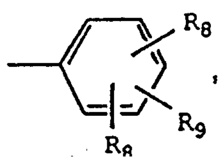 , et

$R_9$ représente un atome d'halogène, un groupe CN, un groupe $CO_2R_2$ ou un groupe $OR_{10}$.

5. Composé selon l'une quelconque des revendications 1 à 4 pour l'utilisation comme produit pharmaceutique.

6. Composé selon la revendication 5 pour l'utilisation dans le traitement du diabète de type II.

7. Composé selon la revendication 5 pour l'utilisation dans le traitement de l'obésité.

8. Composé selon la revendication 5 pour l'utilisation dans l'agonisation du récepteur $\beta_3$.

9. Formulation pharmaceutique comprenant comme ingrédient actif un composé selon l'une quelconque des revendications 1 à 4, associé à un ou plusieurs supports, excipients ou diluants pharmaceutiquement acceptables.

10. Procédé de préparation d'un composé répondant à la Formule I qui comprend :

à l'étape 1, la réaction d'un époxyde répondant à la formule :

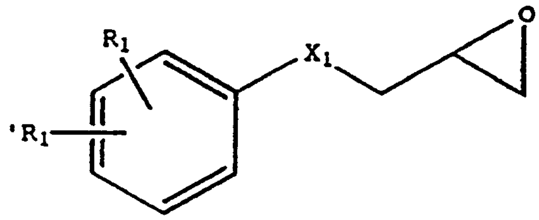

(II)

avec une amine répondant à la formule (B) :

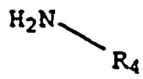

(III) ;

et à l'étape 2, la réaction du produit de l'étape 1 pour former un sel d'addition acide.